# EUROPEAN PATENT SPECIFICATION

(11) **EP 3 125 746 B1**
(45) Date of publication and mention of the grant of the patent: **17.11.2021**
(21) Application number: 15712883.6
(22) Date of filing: 26.03.2015
(51) Int. Cl.: A61B 5/00, A61B 5/024, A61B 5/318

(54) **SYSTEM AND METHOD FOR DETECTING VARIATION OF HEART RATE OF A USER**
SYSTEM UND VERFAHREN ZUR ERKENNUNG DER HERZFREQUENZ EINES BENUTZERS
SYSTÈME ET PROCÉDÉ DE DÉTECTION DE VARIATION DE FRÉQUENCE CARDIAQUE D'UN UTILISATEUR

(30) Priority: 02.04.2014 EP 14163114
(43) Date of publication of application: 08.02.2017
(73) Proprietor: Koninklijke Philips N.V., 5656 AG Eindhoven (NL)
(72) Inventor: COPPOLA, Giuseppe, NL-5656 AE Eindhoven (NL); HUIJBREGTS, Laurentia Johanna, NL-5656 AE Eindhoven (NL)
(74) Representative: Philips Intellectual Property & Standards
(86) International application number: PCT/EP2015/056482
(87) International publication number: WO 2015/150199

(56) References cited:
- WO-A1-2012/140559
- JP-A- H0 871 047
- US-A1- 2006 200 011
- US-A1- 2009 326 356
- US-A1- 2011 245 633
- US-B1- 7 177 686

## Description

### FIELD OF THE INVENTION

The present invention relates to detection of physiological signals of cardiovascular systems. In particular, it relates to a system and method for detecting variation of heart rate of a user. It finds applications in diagnostic investigation of arrhythmias, in particular in the detection of atrial fibrillation. However, it is to be understood that the present invention also finds applications in other fields and is not necessarily limited to the afore-mentioned applications.

### BACKGROUND OF THE INVENTION

Cardiovascular diseases are a class of diseases that involve the circulatory system such as the heart, the blood vessels including arteries, capillaries and veins. Cardiovascular diseases include cardiac diseases, vascular diseases of the brain and kidney, and peripheral arterial diseases. Nowadays, cardiovascular diseases have become one of the leading causes of death for human worldwide. It is thus crucial to develop techniques that enable efficient and accurate detection of symptoms characterizing cardiovascular diseases.

One of the most common groups of conditions belonging to cardiovascular diseases is the arrhythmia, which is characterized by abnormal heart rates. A patient having arrhythmia may have a heartbeat that is too fast or too slow, wherein the heartbeat may be regular or irregular. Arrhythmias can occur in the upper chambers of the heart (atria) or in the lower chambers of the heart (ventricles).

To date, there exist several detection techniques for atrial fibrillation (AF). For instance, a Holter monitor can be worn by a patient for a certain time period, generally between one day and two weeks, in order to monitor the heart activity of the patient continuously. The heart activity is usually characterized by the electrocardiography (ECG), which is able to provide information on the cause of arrhythmias. For instance, an ECG signal measured between two consecutive heart beats in a healthy person differs strongly from that of a person experiencing an AF episode. Other conditions such as brain activity, electroencephalography (EEG) or arterial pressure may also be monitored. An alternative technique is also based upon measurement of the ECG, wherein a stick to be held with both hands is applied instead of a Holter monitor.

The afore-mentioned techniques are afflicted with a number of drawbacks. For instance, the Holter monitor of the first technique usually comprises several electrodes which need to be contacted with several body parts of the patient, rendering this technique rather obtrusive. The second technique, though being less obtrusive compared to the first one, does not provide a continuous detection but only measures the ECG when the patient is holding the stick with both hands. An enforced continuous measurement may cause significant inconveniences to the patient's daily life activities. Since ECG requires a measurement with at least two electrodes, arranged on both sides of the heart, there are no solutions yet for really unobtrusive ECG measurements.

Besides measuring the patient's ECG, optical sensors can also be applied to detect optical signals which allow to deduce whether the patient shows an AF episode. In particular, such optical sensors may be configured to detect photoplethysmography (PPG) signals, thus providing an unobtrusive method to detect arrhythmias. Such optical sensors may be integrated into a device, in particular a wearable device that can be worn on one or more body parts of the patient, thereby combining the unobtrusiveness and continuous detection. This technique is configured to measure the pulse instead of the electrical signal of the heart, thus providing rather limited information on the precise origin of possible arrhythmias, in particular compared to an ECG measurement. Next to that, movement of the patient may lead to motion artifacts in the PPG signal causing usually a lower signal-to-noise ratio than for corresponding ECG measurements. This may lead to an incorrect derivation of heart rate from the PPG signal.

Another optical method to measure heart rate is by laser speckle. This technique suffers from the same drawbacks as mentioned for PPG in the previous paragraph.

US 2012/0310100 A1 discloses systems and methods for detecting and monitoring arrhythmias from a signal, comprising a signal processing system configured to transform a signal using wavelet transformation and analyze changes in features of the transformed signal to detect pulse rhythm abnormalities. In an embodiment, the system disclosed therein may detect pulse rhythm abnormalities by analyzing energy parameters, morphology changes and pattern changes in the scalogram of a photoplethysmography signal.

WO 2012/140559 discloses a method and a system for triggering the measurement of electrocardiogram (ECG) signal of a user. The system includes a SₚO₂ measuring unit and an ECG measuring unit both embedded in a wrist-mounted device worn by the user. The method including the steps of: continuously measuring SₚO₂ at the wrist of the user, detecting an irregular heart condition from the SₚO₂ measurement, notifying the user to perform an ECG measurement, and initiating the ECG measurement at least partially at the wrist.

### SUMMARY OF THE INVENTION

It is an object of the present invention to provide a system and a method which enable detection of variation of heart rate of a user, which is more accurate, especially when there is doubt on the normalcy of the heart rate, while being user-friendly.

In a first aspect of the present invention, a system for detecting variation of heart rate of a user is presented comprising an optical sensing unit for measuring a heartbeat-related optical signal of said living being over time in a first sensing mode at a first sampling rate, a processing unit for deriving an HR-variation signal from said heartbeat-related optical signal and an analyzing unit for comparing said derived HR-variation signal to a reference HR range. The initiating unit is configured to control the optical sensing unit to switch from a first sensing mode, in which the optical sensing unit has already performed an optical measurement of the user, to a second sensing mode with a second sampling rate which corresponds to a higher sampling rate than the first sensing rate if the analyzing unit has detected that the HR-variation signal in the first sensing mode is not within the reference HR range. The initiating unit initiates a process for measuring an ECG signal of said user if the analyzing unit has detected that the HR variation signal in the second sensing mode is not within the reference HR range.

Therefore, the system is suitable for detection of HR-variation in two stages: in a first stage, the detection is based on an optical measurement while in a second stage the detection is based on an electrical measurement. Such a combination of optical with electrical measurement provides a user with both the unobtrusiveness of optical measurements and the high accuracy of electrical measurements capable of providing the user with information of the actual cause of abnormal behaviors appearing in the detection of the first stage. Once the HR-variation signal detected in the first stage indicates a possible AF episode, the second stage may be initiated. In this way, the duration of obtrusive electrical measurements may be reduced to episodes in which the normalcy of the heart rate is suspicious. Furthermore, during the first stage, the optical measurement may result in arrhythmia-suspicious data, which in reality are caused by insufficient measurement accuracy. In this case, the sensitivity of the optical sensing unit is improved by increasing the sampling rate.

The present invention is thus advantageous over a detection technique based solely upon either optical or electrical measurements. In particular, it is advantageous over the optical technique disclosed by the afore-mentioned prior art.

In an embodiment of the invention, the initiating unit is configured to initiate a process to measure the ECG signal when the derived HR-variation signal deviates from the reference HR range by a pre-determined amount. The pre-determined amount may depend on at least one of the user's demographic information, the user's medical history, one or more of the user's physiological signals and the activity the user is performing.

In another embodiment, the system comprises an electrical sensing unit which is advantageously an ECG unit. This embodiment initiates and/or changes the setting of the second stage of detection, in an automatic manner, when a pre-determined criterion as defined in claim 1 is fulfilled. Since each user may have his/her individual condition, individually different criteria might be used for the sake of detection accuracy. Hence, this embodiment advantageously allows the operation of the device to be adapted to the individual condition of the user. Advantageously, the initiating unit is configured to activate a setting, in particular a user-specific setting, of the electrical sensing unit based on the optical measurement. In this way, the device is able to cause an accurate electrical measurement.

In another embodiment, the optical sensing unit comprises one or more photoplethysmography sensing unit. Such a PPG sensing unit can either be optimized to measure heart rate, in which case it advantageously uses green light, or it can be optimized to measure SₚO₂ (pulse oximeter oxygen saturation), in which case it advantageously uses red and infrared light. One or more photo detectors catch the light that has travelled from the one or more light sources (advantageously LEDs) through the skin towards the photo detector(s) and in that way produce a signal that has a relation to the heart rate. To correct for motion artifacts, the PPG sensing unit advantageously also contains a sensor for noise cancellation, like an accelerometer and/or the addition of light of a different color measured by either yet another or an already used photo detector. A processing unit derives the interbeat intervals (IBI's, where an IBI is the time in between two consecutive heart beats) from the sensor signals of the PPG sensing unit. The processing unit can either physically be integrated in the same device as the PPG sensing unit, or it can be a separate unit. Apart from deriving the IBI's, it might also derive a quality indicator, which is an indicator for the probability that the derived IBI is correct and is therefore related to the signal-to-noise ratio of the motion-corrected PPG signal.

Additional parameters such as the respiration and/or the hypo- and/or hypervolemia may be detected besides the heartbeat-related optical signal. An elaborate analysis of the user's condition is thus obtainable using one single device.

In another embodiment, the system further comprises a user interface element. This embodiment advantageously enables interaction between the device and the user, so that an optical measurement and/or an electrical measurement may be conveniently initiated and performed. Advantageously, the user interface element comprises a graphic user interface (GUI), for example in the form of a touch screen, which enables easy device-user-interaction.

In another embodiment, the system comprises an electrical sensing unit to measure a heartbeat-related electrical signal of the user.

Advantageously, the present invention enables an optical measurement and an electrical measurement of HR-variation using one single system, thus combining the advantages of both measurement techniques. These may be chosen depending upon the user's actual need in order to minimize unnecessary obtrusive detection as well as to maximize the accuracy of the HR-variation detection.

In another embodiment, the electrical sensing unit comprises an electrocardiography ECG unit comprising at least a first and a second electrode, the heartbeat-related electrical signal comprising an ECG signal. Advantageously, this embodiment exploits the well-established ECG technique which is able to provide deep insights into the cardiac cycle and information regarding origin of anomalies in the HRV detected optically. In particular, the ECG technique enables a noninvasive method to detect events occurring within the circulatory system such as the heart. Advantageously, the system comprises a PPG unit and an ECG unit. The present embodiment thus is able to combine the afore-mentioned strengths of both PPG and ECG. In order to measure ECG, the first and second electrodes are separately contacted or contactable to two body parts on sides lying opposite one another with respect to the heart of the user.

In another embodiment, the device further comprises a housing wearable on one or more body parts of the user. It is noted that the term "wearable" shall also include the general meaning of the term "portable". The one or more body parts include all body parts of the human body appropriate for wearing or porting the device, such as the arm, the wrist, the waist, the back, the neck, the leg, the foot, etc. Alternatively, the housing of the device may be portable in a pocket of a garment or a hand bag, while the optical sensing unit comprises at least one optical sensor which is in direct contact with one or more body parts of the user. Advantageously, such a device enables continuous detection of the HR-variation, independent upon the location (at home, in a vehicle/train/aircraft, at working place) and activity (working, resting, doing sport, having a meal) of the user. By wearing the device for a long period of time, a long term study of the HR progressing can be realized which may provide the surgical person with accurate information about the development of heart diseases including AF and other types of arrhythmias. Besides, AF-episodes might occur on any time of the day; carrying the device continuously thereby makes it possible to detect even unexpected AF-episodes.

Advantageously, the device comprises a wristwatch-like device, the housing being wearable on one wrist or arm of said user, containing both an optical sensing unit and two electrodes for an ECG-measurement, where one of the two electrodes needs to be touched by a finger of the other hand for an actual ECG measurement. This advantageously combines an optical sensing unit such as a PPG unit and an electrical sensing unit such as an ECG unit within one system that can be particularly easily worn. Normally, a user is used to wearing/porting a wristwatch, which has little impact on the user's daily activities. Preferably, the first electrode is arranged on a first surface of the housing in contact with the wrist wearing the housing and the second electrode is arranged on a second surface of the housing or connected to the housing via connection means. In this way, an electrical measurement can be particularly easily carried out, since the first electrode is already in constant contact with one of the user's wrists so that the user wearing the device only needs to bring one finger of his/her second hand into contact with the second electrode. The second surface may be facing the user's face so that it is particularly easy for the user to locate. A second electrode that is connected to the device body via connection means such as a cable provides the possibility of ECG measurements on two body parts distantly spaced from each other, such as the hands and the legs.

In another embodiment, the device comprises a communication device. The communication device includes mobile devices such as smartphones, tablets, laptops, watches capable of communication, navigation systems, mobile medical devices, etc. This embodiment advantageously enables to combine the functionality of a medical device with that of a communication device. The two electrodes may be arranged on a surface of the communication device. For instance, in case of a smartphone an electrical detection of HR-variation can be easily performed by holding the smartphone with two hands. This is of particular importance, since smartphones have become one of the most used devices, so that a user using such a smartphone according to the present invention is able to frequently perform optical and/or electrical measurements of HR. In another embodiment, the mobile communication device being a smartphone cooperates with a software program in the form of an app, which contains instructions how to perform the optical and/or electrical measurements as well as how to retrieve user information such as patient history and/or patient specific settings for the afore-mentioned measurements.

In a further aspect of the present invention, a method as defined in claim 12 is provided.

In yet further aspects of the present invention, there are provided a computer program which comprises program code means for causing a system as disclosed herein to perform the steps of the method disclosed herein when the computer program is carried out on the system, as well as a non-transitory computer-readable recording medium that stores therein a computer program product, which, when executed by the system, causes the method disclosed herein to be performed.

### BRIEF DESCRIPTION OF THE DRAWINGS

These and other aspects of the invention will be apparent from and elucidated with reference to the embodiment(s) described hereinafter. In the following drawings:
Fig. 1 shows a schematic block diagram of a device in accordance with a first embodiment;
Fig. 2 shows a schematic block diagram of a system in accordance with a first embodiment;
Fig. 3 shows a side view of a system in accordance with a second embodiment;
Fig. 4A and Fig. 4B show a front and a back view of the system in accordance with the second embodiment shown in Fig. 3, respectively;
Fig. 5A and Fig. 5B shows a front and a side view of a system in accordance with a third embodiment, respectively
Fig. 6 shows a schematic block diagram of a method for detecting HRV of a living being;
Fig. 7 shows a schematic block diagram of a method in accordance with the invention when the optical sensing unit determines an arrhythmia-suspicious period; and
Fig. 8 is a schematic graph showing how to determine whether the measured instantaneous heart rate is arrhythmia suspicious.

### DETAILED DESCRIPTION OF THE INVENTION

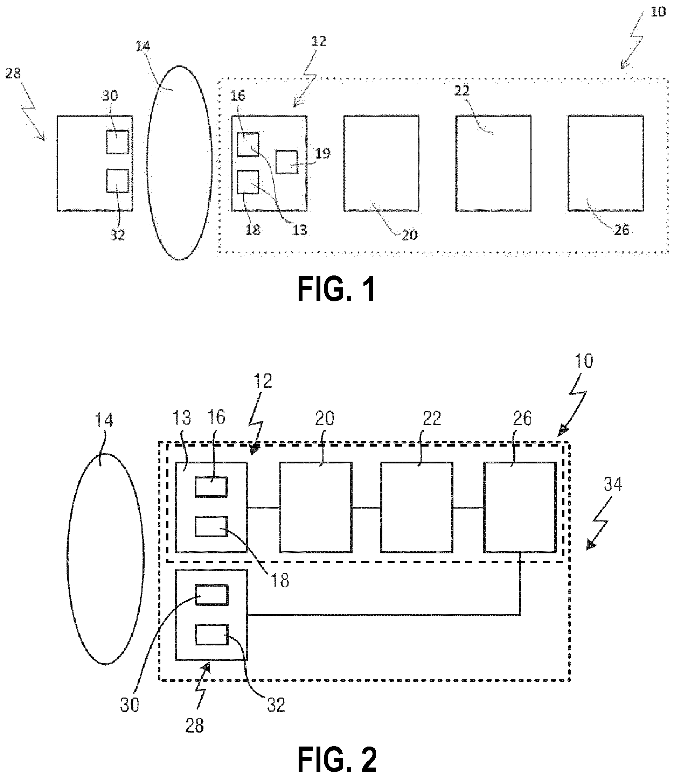

With reference to Fig. 1 a schematic block diagram of a device 10 for detecting heart rate variation in accordance with a first embodiment is shown. The device 10 comprises an optical sensing unit 12 such as an optical sensor for measuring a heartbeat-related optical signal of a living being 14 over time, the living being the user of the device 10. Such optical sensor units are known in the art. A possible optical sensor will be briefly described now. Note that other optical sensors are also possible in relation to the invention. As shown in Fig. 1, the optical sensing unit 12 may comprise an emitter 16 (for example an LED) for emitting light to the living being 14 and a photo detector 18 (like a photodiode) to detect the light that has travelled from the emitter 16 through the living body 14 to the photo detector 18. The optical sensing unit 12 may also comprise more than one light emitters 16 and/or more than one photo detectors 18. The light emitters may or may not have the same color. When the optical sensing unit 12 is optimized for heart rate measurements, the one or more light emitters 16 advantageously emit green light. When the optical sensing unit 12 in optimized for pulse oximeter oxygen saturation SₚO₂ measurements, it contains advantageously one or more red light emitters and one or more infrared light emitters. The combination of one or more light emitters 16 and one or more photo detectors 18 meant for measuring the PPG signal together form the optical sensor 13. To correct for motion artifacts, the optical sensing unit may also contains an additional sensor 19 for noise cancellation, like an accelerometer and/or the addition of light of a different color measured by either yet another photo detector or a one or more photo detectors 18 that are also used in the optical sensor 13.

A processing unit 20 such as a processor derives the interbeat intervals (IBI's, where an IBI is the time in between two consecutive heart beats) from the sensor signals of the optical sensor 13 and the noise-cancellation sensor 19 of the optical sensing unit 12. Apart from deriving the IBI's, the processing unit 20 might also derive a quality indicator, which is an indicator for the probability that the derived IBI is correct and is therefore related to the signal-to-noise ratio of the motion-corrected PPG signal. Instead of deriving IBI's (with the unit of time, like seconds or milliseconds), the processing unit 20 may also derive the frequency of heartbeat signals, so that it is a continuous function of time representing the momentary value of heartbeat frequency having unit of hertz (Hz) or kilohertz (kHz). We will use the term instantaneous heart rate for this value, although it must be noted that in this case it is derived from the PPG signal and not from the RR-peak in the ECG, as it is normally done. It is not necessarily needed that the processing unit 20 derives the power spectra from the instantaneous heartbeat or the IBI's, as is normally done for heart rate variability (HRV). For this reason, we use the term HR-variation signal instead of HRV to cover a broader range, in which not necessarily the power spectra of the high frequency band, low frequency band and/or ultralow frequency band are calculated, but which also reflects the consecutive IBI's or instantaneous heart rates.

In the embodiment shown in Fig. 1, the device 10 further comprises an analyzing unit 22 such as a comparator for comparing the HR-variation signal derived by the processing unit 20 to a reference HR range. In one embodiment, the reference HR range may be a pre-determined HR range corresponding to the range of IBIs of a healthy person. Alternatively, the analyzing unit 22 may compare the individual IBIs determined from the measured heartbeat-related optical signal of the patient to the individual IBIs of a healthy person, in particular a person without arrhythmia or atrial fibrillation AF but showing the same average HR as the patient.

In the embodiment shown in Fig. 1, the device 10 further comprises an initiating unit 26 such as a controller for initiating an electrical sensing unit 28, said electrical sensing unit 28 being configured to measure a heartbeat-related electrical signal of the living being 14. The electrical sensing unit 28 comprises a first electrode 30 and a second electrode 32, the first and/or the second electrodes 30, 32 being advantageously contactable to the living being 14. The initiating unit 26 may be configured to activate the electrical sensing unit 28 when the HR-variation signal derived by the processing unit 20 deviates from the reference HR range, in particular by a pre-determined amount.

In the embodiment shown in Fig. 1, the electrical sensing unit 28 is a separate unit from the device 10. The electrical sensing unit 28 may be an electrocardiography ECG sensor, the heartbeat-related electrical signal comprising an ECG signal.

In the embodiment shown in Fig. 1, the optical sensing unit 12 is arranged on one side of the living being 14. It is understood that the optical sensing unit 12 may be arranged to be in the vicinity of any other side(s) of the living being 14. In particular, a plurality of optical sensors 13 may be arranged at different positions that are appropriate for performing an optical measurement of the living being 14.

In the embodiment shown in Fig. 1, the electrical sensing unit 28 comprises only two electrodes 30, 32. It is understood that the electrical sensing unit 28 may comprise further electrodes and that all the electrodes of the electrical sensing unit 28 may be arranged at least partially at different positions with respect to the living being 14 that are appropriate for performing an electrical measurements of the living being 14.

In the embodiment shown in Fig. 1, the processing unit 20, analyzing unit 22, and initiating unit 26 are integrated in the same device 10 as the optical sensing unit 12. Although the word unit is used, the processing unit 20, analyzing unit 22, and initiating unit 26 need not necessarily to be separate physical entities but can be a single physical entity such as a processor. The processing, analyzing and/or initiating functions can also be in the form of a code which is executed by the same computer program.

In another embodiment, the optical sensing unit 12, the processing unit 20, analyzing unit 22 and /or initiating unit 26 are integrated in one or more different devices, using a wired or wireless connection between each other (the processing unit 20 should at least be in connection with the optical sensing unit 12, the analyzing unit 22 should be in connection with the processing unit 20, and the initiating unit 26 should be in connection with the analyzing unit 22). A possibility is to use a smart phone as separate device, containing one or more processing unit 20, analyzing unit 22, and initiating unit 26.

In another embodiment, the user may wear the optical sensing unit 12 on his/her wrist and an electrical sensing unit 28 in his/her pocket or hand bag. The analyzing unit 22 can be integrated in the same device as the optical sensing unit 12 or the electrical sensing unit 28 ore may be a separate device in wireless connection with (at least) the optical sensing unit 12. If the analyzing unit 22 detects an arrhythmia-suspicious period, the user is warned and thereby asked to take the electrical sensing unit 28 from pocket / hand bag and perform an electrical measurement, e.g. an ECG measurement.

In the embodiment shown in Fig. 1, the initiating unit 26 initiates an electrical measurement. Here the word initiating includes but is not limited to one or more of the following options: automatically start an electrical measurement (irrespective of whether or not the user is already connected to the electrodes), automatically start the recording of the electrical measurement (to enable the user to look back the data later), mark / time stamp the electrical measurement data that are being recorded (to enable the user to easily find back the valuable data), let the user know that he/she should connect to the electrodes to do an ECG measurement (for example with text or images on a display, with a recorded or simulated voice, or with other , visual, auditory or vibrational signs known by the user).

With reference to Fig. 2, a schematic block diagram of a system 34 in accordance with a first embodiment is shown.

The system 34 comprises a device for detecting HR-variation of a living being, advantageously the device 10 shown in Fig. 1. The system 34 further comprises an electrical sensing unit to measure a heartbeat-related electrical signal of the living being 14. Advantageously, the system 34 comprises the electrical sensing unit 28 described with reference to Fig. 1.

When using the device 10 in Fig. 1 and 2, the emitter 16 emits light which propagates to one or more body parts of the living being 14. Advantageously, the emitter 16 emits light of red light wavelength and IR light wavelength to one or more body parts, for example the skin of the living being 14. The detector 18 detects the light which emanates back from the living being 14 after being emitted by the emitter 16. In this way, the optical sensing unit 12 performs measurement of a heartbeat-related optical signal. The heartbeat-related optical signal measured by the optical sensing unit 12 is then processed by the processing unit 20 to generate an HR-variation signal. In particular, the heartbeat-related optical signal comprises a plurality of heartbeat signals, wherein adjacent heartbeat signals are separated by interbeat intervals (IBI). By measuring the heartbeat-related optical signal for a certain time period, a certain number of heartbeat signals and consequently a certain number of IBI may be measured. The processing unit 20 thus derives an HR-variation signal from the measured heartbeat-related optical signal by calculating the plurality of IBIs.

In an example useful for understanding the invention, the analyzing unit 22 then compares the HR-variation signal derived by the processing unit 20 to the reference HR range as described above. If the derived HR-variation resides within the reference HR range, the optical measurement indicates that the living being 14 shows a normal heart rate behavior. If the derived HR-variation signal deviates from the reference HR range by a pre-determined amount, the optical measurement suggests that the living being shows an abnormal heart rate behavior and further investigation of the heart rate condition of the living being 14 may be necessary. In this case, the initiating unit 26 of the device 10 shown in Fig. 1 may activate the electrical sensing unit 28 that is either arranged externally from the device 10 or in combination with the device 10 within the system 34 to perform an electrical measurement for the living being 14. In particular, the initiating unit 26 may activate the ECG unit 28 to measure an ECG signal of the living being 14. Moreover, the initiating unit 26 may control the optical sensing unit 12 to switch from a first sensing mode, in which the optical sensing unit 12 has already performed an optical measurement of the living being 14, to a second sensing mode, wherein the second sensing mode corresponds to a higher sensitivity than the first sensing mode. In particular, the second sensing mode corresponds to a higher sample rate than the first sensing mode. The initiating unit 26 may also control an alerting unit (not shown) to send one or more alert signals. The one or more alert signals may be visual and/or acoustical and/or vibrational signals. For instance, the one or more alert signals may comprise a flashing light signal, appearing in the vicinity of at least one electrode of the ECG unit 28, and/or a voice signal indicating that an HR-variation signal deviating from the reference HR range has been detected and an electrical measurement may be performed and/or a vibration of one or more parts of the device 10. In a preferable example, the initiating unit 26 activates the electrical sensing unit 28 after one or more alert signals have been sent by the alerting unit and after the user has input a command to the system 34 and/or the device 10 and/or the electrical sensing unit 28. In another preferable example, the initiating unit 26 switches the optical sensing unit 12 from the first to the second sensing mode after the one or more alert signals have been sent and the user has input a command to the system 34 and/or the device 10 and/or the optical sensing unit 12.

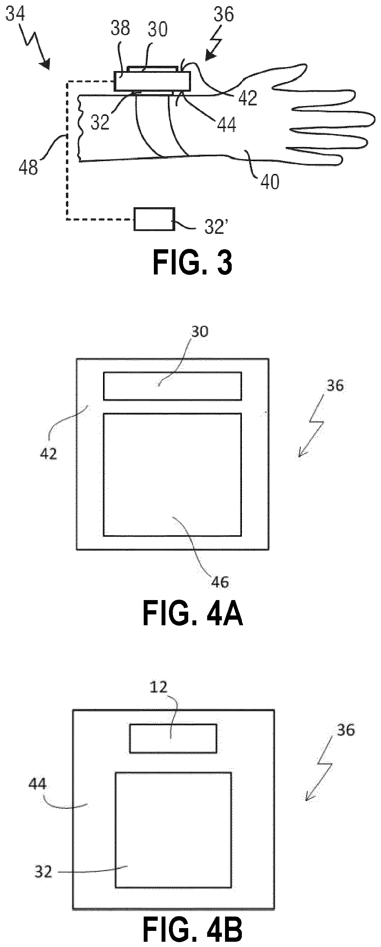

With reference to Fig. 3, a system 34 in accordance with a second embodiment is shown. The system 34 comprises a wearable device 10, wherein the wearable device 10 comprises a housing 36 that can be worn by the living being 14. In particular, the system 34 comprises a wristwatch-like device 36 with a housing 38 wearable on one wrist or arm of the living being 14. The first electrode 30 of the electrical sensing unit 28 is arranged on a first surface 42 of the housing 38, whereas the first surface 42 is not in contact with the wrist or arm 40 of the living being 14. The second electrode 32 of the electrical sensing unit 28 is arranged on a second surface 44 of the housing 38. The second surface 44 may be opposite to the first surface 42 with respect to the housing 38, so that the second electrode 32 may be configured to be in permanent contact with the skin of the wrist or arm 40.

In a preferable embodiment, one or more optical sensors 13, in particular PPG sensors, may be arranged on the second surface 44 so that they may measure a heartbeat-related optical signal, in particular a PPG signal of the living being 14. The PPG signal may be measured directly from the wrist or arm of the living being 14.

In Fig. 4A and B, the wristwatch-like device 36 of Fig. 3 are shown in a front and a back view, respectively. In a preferable embodiment, the wristwatch-like device 36 further comprises a user interface element 46, in particular a graphic user interface (GUI) element 46, which enables an interaction between the system 34 and the user. In particular, the GUI element 46 may be configured to show the heartbeat-related optical signal measured by the optical sensing unit 12 and/or the HR-variation signal derived by the processing unit 20 and/or the reference HR range and/or the heartbeat-related electrical signal measured by the electrical sensing unit 28 and/or the one or more alert signals sent by the alerting unit. Also an average heart rate (average over at least several seconds, having the unit beats per minute) could be calculated from the PPG or ECG signal and shown on the GUI element 46. Another option is that the GUI element 46 shows information not related to the measurements of the heart, for instance the time, by which device 36 can be used as a normal watch, or internet, SMS (Short Message Service) etc, by which it can be used as a smart watch. The GUI element 46 may be a cathode ray tube monitor, a flat panel display, a liquid crystal display (LCD), a plasma display or any other type of monitor or display known in the art. The GUI element 46 may cooperate with a GUI program based on Windows, Linux, MS OS, KDE and any suitable GUI program known in the art.

By placing first electrode 30 not on the front side 42, but on the side of the watch or on the strap instead, the GUI element 46 can cover the whole front side 42 of the watch.

The optical measurement of the heartbeat-related optical signal of the living being 14 can be performed once the user has put on the wristwatch-like device 36. It is understood that the wearable device 36 may also be configured to be worn at another body part, such as the forehead, the neck, the waist, the leg and/or the foot of the living being 14. After the optical sensing unit 12 of the wristwatch-like device 36 has detected an HR-variation signal of the living being 14 and that the derived HR-variation signal has been found to deviate from a pre-determined reference HR range, in particular by a pre-determined amount, the GUI element 46 displays an alert signal, advantageously a text message informing the user about the result of the optical measurement and/or instructing the user to start an electrical measurement. Alternatively, the GUI element 46 may display an alert signal comprising a text message instructing the user to stop moving, to attach the device with the optical sensor 13 properly, and/or to increase the sample rate of the optical sensing unit 12. Another possibility is that the device 36 automatically increases the sample rate of the optical sensing unit 12. After a message instructing the user to start an electrical measurement, by placing one or more fingers of the other hand not wearing the wristwatch-like device 36 onto the first electrode 30, an ECG signal will be measured. Advantageously, the data captured by the ECG unit will be stored in a memory unit. In that way, a doctor could examine the data later to make an analysis of the arrhythmia. The memory unit may be integrated in the system 34. Alternatively, the system 34 may comprise a data interface with which data can be send to and/or are retrievable from an external data base, such as a network system, a cloud system, the internet, an intranet, a personal computer or a mobile communication device including a smartphone, or any other mobile devices. The data interface may be based on USB, Blue Tooth, optical fiber or other data interface technologies known in the art.

Alternatively, the electrical sensing 28 of the system 34 may comprise a second electrode 32' which is connected to the housing 38 of the wristwatch-like device 36 via connection means 48. In particular, the connection means 48 may comprise a cable 48. The wired second electrode 32' may be brought into contact with a different body part of the living being 14 such as the chest, the neck, the forehead, the face, the arm, the wrist, the back, the leg, the feet, etc. By bringing the wired second electrode 32' to the hand or the arm or the wrist not wearing the wristwatch-like device 36, an electrical measurement of the HR-variation signal may be performed with two body parts on size lying opposite one another with respect to the heart of the living being 14. As an ECG with even more than two electrodes could be valuable, there is also the possibility to have even more electrodes. In a preferable embodiment, it is easy to attach and detach the one or more cables 48 of the one or more electrodes 32' to/from the device to keep the device 36 unobtrusive. When the initiating unit 26 warns the user to do an ECG measurement, he/she should then attach the one or more cables 48 to the device and the one or more electrodes 32' to the desired place(s) on the body. In this embodiment, the electrode 30 on top of the device and/or electrode 32 on the bottom of the device may be absent.

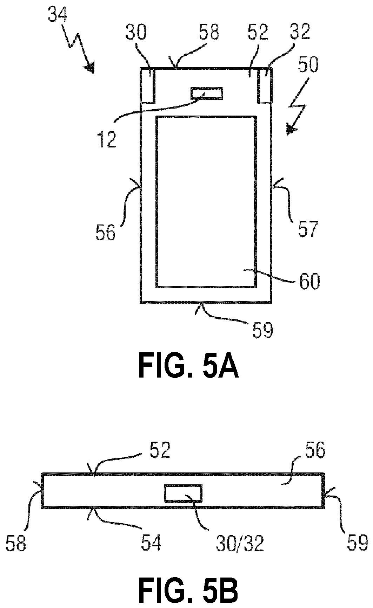

With reference to Fig. 5A a system 34 in accordance with a third embodiment is shown in a front view.

In this embodiment, the device 10 comprises a mobile communication device 50, in particular a smartphone. Alternatively, the mobile communication device 50 may be configured as a tablet, a calculator, a portable media player, a video camera or a navigation device. The first and second electrodes 30, 32 of the electrical sensing unit 28, in particular being an ECG unit, are advantageously arranged on a front surface 52 of the mobile communication device 50. Alternatively, the electrical sensing unit 28 may comprise further electrodes, wherein at least two of all electrodes of the electrical sensing unit 28 are arranged on two different surfaces of the mobile communication device 50. In particular, at least one electrode 30, 32 may be arranged on a back surface 54 or a side surface 56, 57, 58, 59, as shown in Fig. 5B.

The optical sensing unit 12 is arranged advantageously on the front surface 52, whereas it can be alternatively arranged on the back surface 54 or one of the side surfaces 56, 57, 58, 59. In a preferable embodiment, the optical sensing unit 12 comprises a plurality of optical sensors 13 which are arranged at least partially on different surfaces 52, 54, 56, 57, 58, 59. Alternatively, the optical sensing unit 12 is a camera. Nowadays, most smart phones already have a standard camera. Together with a dedicated app, the information from the camera can be used to derive the heart rate.

The mobile communication device 50 further comprises a display unit 60, the display unit 60 advantageously comprising a touchscreen. Advantageously, a GUI element such as the GUI element 46 is integrated into or configured to cooperate with the display unit 60 of the mobile communication device 50.

In an example useful for understanding the invention, a computer program such as an app may be configured to cooperate with the mobile communication device 50. After starting the app via the display unit 60, the user may start an optical measurement of the HR-variation signal. When the optical sensing unit 12 detects an HR-variation signal deviating from a reference HR range by a pre-determined amount, the display unit 60 displays an alert message and provides the user with several options containing: increasing the sample rate of the optical sensing unit 12; proceeding with an electrical measurement of the HR-variation signal; consulting a doctor/a surgical person/a care giver, etc. Each of the afore-mentioned options may be associated with a visual button shown on the display unit 60, by clicking of which the chosen option will be processed. For instance, if the user clicks the visual button associated with the option "increasing the sample rate of the optical sensing unit", the display unit 60 displays a visual control panel for the optical sensing unit 12 through which the sample rate of at least one optical sensor 13 of the optical sensing unit 12 may be varied. If the user choses the option "proceeding with an electrical measurement", the display unit 60 displays an guide message for performing an electrical measurement, advantageously with the electrical sensing unit 28 arranged within the system 34. Alternatively, the guide message may be configured for guiding the user to perform an electrical measurement using an external electrical sensing unit 28, advantageously an external ECG unit connectable to the mobile communication device 50 via a communication link known in the art. If the user choses the option "consulting a doctor", the display unit 60 display a list of contact information, advantageously with feed-back information.

In a preferable embodiment, the optical sensing unit 12 and the electrical sensing unit 28 of the system 34 according to one or more embodiments shown above may be operated simultaneously. In another preferable embodiment, the system 34 and/or the device 10, 36, 50 may communicate with an external database, an external network system such as the internet, an intranet, a cloud system or a PC or another mobile communication device either using a wired communication link or wirelessly.

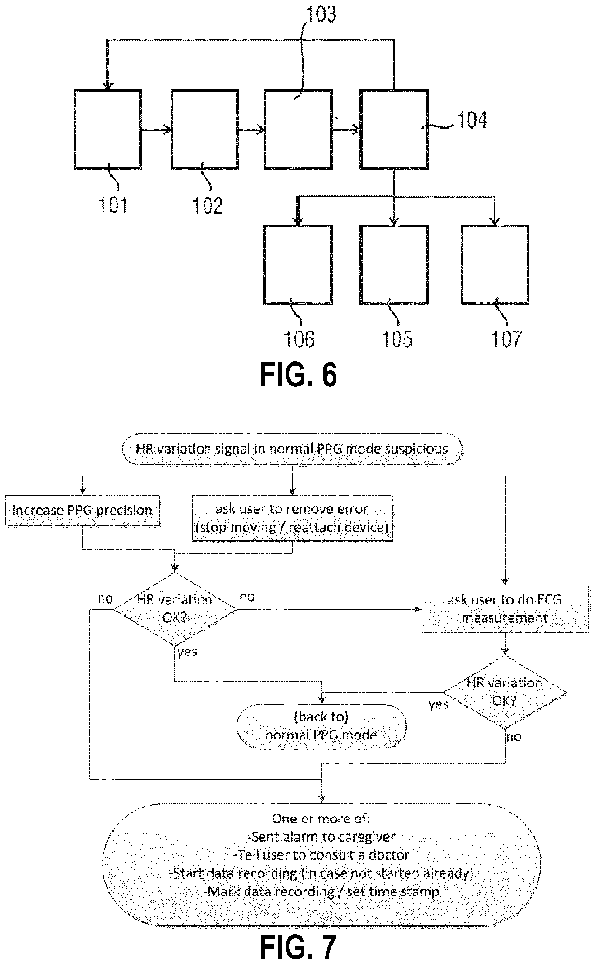

With reference to Fig. 6 , according to an example useful for understanding the present invention, a method for detecting HRV of a living being 14 is shown. In a step 101, a heartbeat-related optical signal of the living being 14 is measured over time. This can be done by using the device 10, 36, 50 according to the afore-mentioned embodiments. The heartbeat-related optical signal is then proceeded to derive an HR-variation signal in step 102. In particular, the heartbeat-related optical signal comprises a plurality of heartbeat signals, wherein a plurality of interbeat intervals (IBI) can be derived. The derived HR-variation signal is then compared to a reference HR range in a step 103, wherein the reference HR range is advantageously a pre-determined HR range based on HR-variation signal measured for a healthy living being. In a step 104, the result of the comparison in the step 103 is provided to the initiating unit 26 for controlling the electrical sensing unit 28, in particular being an ECG unit. In a step 105, the initiating unit 26 activates the electrical sensing unit 28 to perform a measurement of a heartbeat-related electrical signal, once the derived HR-variation signal has been found to deviate from the reference HR range by a pre-determined amount. Alternatively, the electrical sensing unit 28 has already been activated previously at the time of the comparison in step 103, while step 105 can for example be starting recording the ECG data and/or showing the ECG signal on a display. Recording the ECG data (either or not only in a period with arrhythmia-suspicious PPG data) is in any case a desirable function of the device, because then the doctor would be able to look back the shape of the ECG waveform, to make conclusions on the origin of the arrhythmias. To easily find back the point where the PPG data became arrhythmia suspicious, apart from activating the electrical sensing unit and/or starting the ECG recording and/or showing the ECG signal on a display, step 105 could thus also involve marking the trace of ECG data and /or PPG data that are being recorded.

The method comprises a step 106 in which the initiating unit 26 switches the optical sensing unit 12 from a first to a second sensing mode wherein the second sensing mode corresponds to higher sensitivity than the first sensing mode. The method comprises a step 107, in which one or more alert signals are sent. The alert signals may contain visual acoustic and/or vibrational signals. In a preferable embodiment the alert signals contain a text message for instructing the user to choose between different options as described above with reference to Fig. 5A.

Fig. 7 shows a schematic block diagram of possible steps when the PPG sensing unit determines an arrhythmia-suspicious period. When the PPG sensing unit determines an arrhythmia-suspicious period, according to the present invention, the precision of the PPG sensing unit is increased by increasing the sample rate. Alternatively, not covered by the present invention, the user can be asked (by a text on a display, by an audio-recorded/simulated voice, or by other visual, vibrational, or auditory signs known to the user) to correct a measurement error (e.g. by stop moving or improve the contact of the optical sensor with the skin, for example by tightening or reattaching the device with the optical sensing unit) or to start an electrical measurement. If after correcting the measurement error or, according to the present invention, increasing the sensitivity mode of the PPG sensing unit, the PPG signal is still arrhythmia suspicious, the user can be asked to start an electrical measurement or directly an alarm can be sent out to a caregiver and/or feedback is given to the user that he/she should consult a doctor and/or the data could be recorded/marked to be looked back later by the doctor. The latter three could also be the next step when the ECG turns out to indicate a period of arrhythmia. If either the error-corrected/increased-precision PPG measurement or the ECG measurement shows a normal heart-rate variation signal, the mode can go back to the normal PPG measurement mode.

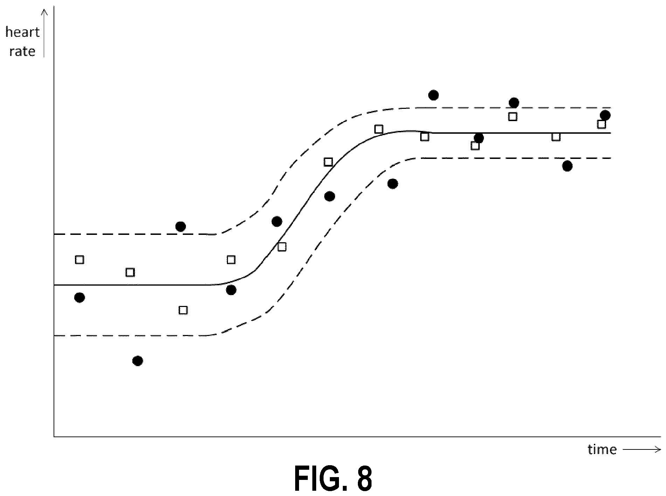

Fig. 8 shows a schematic graph of an example of how to determine whether the measured instantaneous heart rate is arrhythmia suspicious. The solid line shows the average heart rate (averaged over at least a couple of seconds) of a person. At a certain time it starts to increase because the person starts to exercise. As boundaries to decide what is a normal heart-rate variation and what is not, the dashed lines are taken. The open squares are the instantaneous heart rates of a healthy person, whereas the dots are the instantaneous heart rates of a person with arrhythmias. The figure shows that all instantaneous heart rates of the healthy person fall inside the boundaries, while for the person with arrhythmias there are dots that fall outside the boundaries. Therefore, a pattern as shown by the dots, if measured by the optical sensing unit 12, would give rise for initiating unit 26 to initiate one of the afore-mentioned processes, like telling the user to start an electrical measurement. It is also possible to give the user continuous feedback on heart-rate variation OK or heart-rate variation not OK based on whether or not the heart rate falls inside the boundaries. This could be the heart rate measured by the optical unit and/or the heart rate measured by the ECG unit. The boundaries could be taken fixed, in the sense that they are always the same amount above and below the average heart rate, or they could be more specific, meaning that the boundaries for example depend on the user's demographic information (like age and gender), on the user's medical history, on one or more of the user's physiological signals (average heart rate, change in average heart rate, SₚO₂, respiration rate, etc.) and/or on the activity the user is performing (sitting, sleeping, walking, running, cycling etc.)

If the device 10 has got the ability to ask the user to stop moving, like in the embodiment of Fig. 8, it might use the signal of the noise-cancellation sensor 19 to determine whether the user is making disturbing movements at all. If the user is indeed making disturbing movements, he/she is asked to stop moving, if not, another option is chosen. Similarly, the device 10 could be made automatic such that the sample rate will not be increased if this is not a limiting factor and/or the user will not be asked to improve the contact of the optical sensor 13 with the skin if the signal-to-noise ratio is already high.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims. For example, although a PPG sensing unit in contact with the skin has been mainly disclosed in the description as being the optical sensing unit, the same invention holds for other optical sensing units, like a laser speckle sensing unit, or optical sensing units not in contact with the skin, like a vital signs camera. Next to that, more than one optical sensing unit may be used.

In the claims, the word "comprising" does not exclude other elements or steps, and the indefinite article "a" or "an" does not exclude a plurality. A single element or other unit may fulfill the functions of several items recited in the claims. The mere fact that certain measures are recited in mutually different dependent claims does not indicate that a combination of these measures cannot be used to advantage.

A computer program may be stored and/or distributed on a suitable medium, such as an optical storage medium or a solid-state medium supplied together with or as part of other hardware, but may also be distributed in other forms, such as via the Internet or other wired or wireless telecommunication systems.

Any reference signs in the claims should not be construed as limiting the scope.

## Claims

1. A system (34) for detecting variation of heart rate, HR, of a user (14), comprising:
- an optical sensing unit (12) for measuring a heartbeat-related optical signal of said user (14) over time in a first sensing mode at a first sampling rate;
- a processing unit (20) for deriving an HR-variation signal from said heartbeat-related optical signal;
- an analyzing unit (22) for comparing said derived HR-variation signal to a reference HR range; and
- an initiating unit (26) configured to control the optical sensing unit (12) to switch from the first sensing mode, in which the optical sensing unit (12) has already performed an optical measurement of the user (14), into a second sensing mode with a second sampling rate which corresponds to a higher sampling rate than the first sensing mode if the analyzing unit (20) has detected that the HR-variation signal in the first sensing mode is not within the reference HR range,
wherein the initiating unit (26) is configured to initiate a process for measuring an ECG signal of said user (14) if the analyzing unit (20) has detected that the HR-variation signal in the second sensing mode is not within the reference HR range.

2. The system (34) according to claim 1, wherein said initiating unit (26) is configured to initiate a process to measure the ECG signal when said derived HR-variation signal deviates from said reference HR range by more than a pre-determined amount.

3. The system (34) according to claim 2, wherein the pre-determined amount depends on at least one of the user's demographic information, the user's medical history, one or more of the user's physiological signals and the activity the user is performing.

4. The system (34) according to claim 1, wherein said optical sensing unit (12) comprises one or more photoplethysmography PPG sensors, said heartbeat-related optical signal comprising a PPG signal.

5. The system (34) according to claim 1, wherein the initiating unit (26) initiates one or more of the following:
- automatically start an ECG measurement,
- automatically start a recording of the ECG measurement,
- mark the ECG measurement data that are being recorded, -
- inform the user that he/she should connect to electrodes of an ECG unit,
- show the ECG signal on a graphic user interface element (46).

6. The system (34) according to claim 1, further comprising a user interface element (46).

7. A system (34) according to claim 1, comprising an electrical sensing unit (28) to measure a heartbeat-related electrical signal of said user (14).

8. The system (34) according to claim 7, wherein said electrical sensing unit comprises an electrocardiography ECG unit comprising at least a first (30) and a second electrode (32), said heartbeat-related electrical signal comprising an ECG signal.

9. The system (34) according to claim 8, comprising a wristwatch-like device (10), said device (10) comprising a housing (38) being wearable on one wrist or arm of said user (14).

10. The system (34) according to claim 9, wherein said first electrode (30) is arranged on a first surface (42) of said housing (38) in contact with said one wrist or arm wearing said housing (38) and said second electrode is arranged on a second surface (44) of said housing (38) or connected to said housing (38) via connection means (48).

11. The system (34) according to claim 1, wherein said device (10) comprises a communication device (50).

12. A method for detecting variation of heart rate, HR, of a user (14), comprising the steps of:
- measuring a heartbeat-related optical signal of said user (14) over time with an optical sensing unit (12) in a first sensing mode at a first sampling rate;
- deriving an HR-variation signal from said heartbeat-related optical signal;
- comparing said derived HR-variation signal to a reference HR range;
- switching from the first sensing mode, in which the optical sensing unit (12) has already performed an optical measurement of the user (14), into a second sensing mode with a second sampling rate which corresponds to a higher sampling rate than the first sampling rate if the comparison of the HR-variation signal in the first sensing mode is not within the reference HR range, and
- initiating a process for measuring an ECG signal of said user (14) if the HR-variation signal in the second sensing mode is not within the reference HR range.

13. Computer program comprising program code means for causing the system according to any of claims 1 to 11 to carry out the steps of the method as claimed in claim 12 when said computer program is carried out on the system.

## Patentansprüche

1. Ein System (34) zum Erkennen von Schwankungen der Herzfrequenz, HF, eines Benutzers (14), das Folgendes umfasst:
- eine optische Sensoreinheit (12) zum Messen eines optischen Herzschlagsignals des Benutzers (14) über die Zeit in einem ersten Erfassungsmodus mit einer ersten Abtastrate;
- eine Verarbeitungseinheit (20) zum Ableiten eines HF-Variationssignals aus dem optischen Herzschlagsignal;
- eine Analyseeinheit (22) zum Vergleichen des abgeleiteten HF-Variationssignals mit einem HF-Referenzbereich; und
- eine Auslöseeinheit (26), die die optische Sensoreinheit (12) so steuert, dass sie vom ersten Erfassungsmodus, in dem die optische Sensoreinheit (12) bereits eine optische Messung des Benutzers (14) durchgeführt hat, in einen zweiten Erfassungsmodus mit einer zweiten Abtastrate umschaltet, in dem die Abtastrate höher ist als im ersten Erfassungsmodus, wenn die Analyseeinheit (20) erkannt hat, dass das HF-Variationssignal im ersten Erfassungsmodus nicht innerhalb des HF-Referenzbereichs liegt,
wobei die Auslöseeinheit (26) einen Prozess zum Messen eines EKG-Signals des Benutzers (14) auslöst, wenn die Analyseeinheit (20) erkannt hat, dass das HF-Variationssignal im zweiten Erfassungsmodus nicht innerhalb des HF-Referenzbereichs liegt.

2. Das System (34) gemäß Anspruch 1, wobei die Auslöseeinheit (26) einen Prozess zum Messen des EKG-Signals auslöst, wenn das abgeleitete HF-Variationssignal um mehr als einen vorgegebenen Betrag vom HF-Referenzbereich abweicht.

3. Das System (34) gemäß Anspruch 2, wobei der vorgegebene Betrag von mindestens einem der folgenden Aspekte abhängig ist: der demographischen Daten des Benutzers, der medizinischen Vorgeschichte des Benutzers, mindestens einem physiologischen Signal des Benutzers und der Aktivität, die der Benutzer ausführt.

4. Das System (34) gemäß Anspruch 1, wobei die optische Sensoreinheit (12) mindestens einen Photoplethysmographie-Sensor (PPG) umfasst,
wobei das optische Herzschlagsignal ein PPG-Signal umfasst.

5. Das System (34) gemäß Anspruch 1, wobei die Auslöseeinheit (26) mindestens einen der folgenden Schritte auslöst:
- automatisches Starten einer EKG-Messung,
- automatisches Starten der Aufzeichnung der EKG-Messung,
- Markieren der gerade aufgezeichneten EKG-Messdaten werden,
- den Benutzer Auffordern, sich an den Elektroden eines EKG-Geräts anzuschließen,
- Anzeigen des EKG-Signals auf einem Element der grafischen Benutzeroberfläche (46).

6. Das System (34) gemäß Anspruch 1, das zudem ein Benutzerschnittstellenelement (46) umfasst.

7. Ein System (34) gemäß Anspruch 1, das einer elektrische Sensoreinheit (28) zum Messen eines elektrischen Herzschlagsignals des Benutzers (14) umfasst.

8. Das System (34) gemäß Anspruch 7, wobei die elektrische Sensoreinheit eine Elektrokardiographie-EKG-Einheit mit mindestens einer ersten (30) und einer zweiten Elektrode (32) umfasst, wobei das elektrische Herzschlagsignal ein EKG-Signal umfasst.

9. Das System (34) gemäß Anspruch 8, das ein einer Armbanduhr ähnliches Gerät (10) umfasst, wobei das Gerät (10) ein Gehäuse (38) umfasst, das am Handgelenk oder Arm des Benutzers (14) getragen werden kann.

10. Das System (34) gemäß Anspruch 9, wobei die erste Elektrode (30) auf einer ersten Oberfläche (42) des Gehäuses (38) in Kontakt mit dem Handgelenk oder Arm steht, an dem das Gehäuse (38) getragen wird, und wobei die zweite Elektrode auf einer zweiten Oberfläche (44) des Gehäuses (38) angeordnet oder über Verbindungselemente (48) mit dem Gehäuse (38) verbunden ist.

11. Das System (34) gemäß Anspruch 1, wobei das Gerät (10) ein Kommunikationsgerät (50) umfasst.

12. Eine Methode zum Erkennen von Schwankungen der Herzfrequenz, HF, eines Benutzers (14), die folgende Schritte umfasst:
- Messen eines optischen Herzschlagsignals des Benutzers (14) über die Zeit mit einer optischen Sensoreinheit (12) in einem ersten Erfassungsmodus mit einer ersten Abtastrate;
- Ableiten eines HF-Variationssignals aus dem optischen Herzschlagsignal;
- Vergleichen des abgeleiteten HF-Variationssignals mit einem HF-Referenzbereich;
- Umschalten vom ersten Erfassungsmodus, in dem die optische Sensoreinheit (12) bereits eine optische Messung des Benutzers (14) durchgeführt hat, in einen zweiten Erfassungsmodus mit einer zweiten Abtastrate, in dem die Abtastrate höher ist als im ersten Erfassungsmodus, wenn der Vergleich ergab, dass das HF-Variationssignal im ersten Erfassungsmodus nicht innerhalb des HF-Referenzbereichs liegt, und
- Auslösen eines Prozesses zum Messen eines EKG-Signals des Benutzers (14), wenn das HF-Variationssignal im zweiten Erfassungsmodus nicht innerhalb des HF-Referenzbereichs liegt.

13. Ein Computerprogramm, das Programmcode umfasst, mit dem das System gemäß einem der Ansprüche 1 bis 11 veranlasst wird, die Schritte der Methode gemäß Anspruch 12 auszuführen, wenn das Computerprogramm im System ausgeführt wird.

## Revendications

1. Système (34) de détection d'une variation de fréquence cardiaque, FC, d'un utilisateur (14), comprenant :
- une unité optique de détection (12) destinée à la mesure d'un signal optique lié au rythme cardiaque dudit utilisateur (14) au cours du temps dans un premier mode de détection à une première fréquence d'échantillonnage ;
- une unité de traitement (20) destinée à la dérivation d'un signal de variation de fréquence cardiaque à partir dudit signal optique lié au rythme cardiaque ;
- une unité d'analyse (22) destinée à la comparaison dudit signal de variation FC dérivé à une plage FC de référence ; et
- une unité de déclenchement (26) conçue pour commander l'unité optique de détection (12) pour passer du premier mode de détection, dans lequel l'unité optique de détection (12) a déjà effectué une mesure optique de l'utilisateur (14), à un second mode de détection à une seconde fréquence d'échantillonnage, laquelle correspond à une fréquence d'échantillonnage supérieure que celle du premier mode de détection lorsque l'unité d'analyse (20) a détecté que le signal de variation FC dans le premier mode de détection n'est pas dans la plage FC de référence, dans lequel l'unité de déclenchement (26) est conçue pour déclencher un processus de mesure d'un signal ECG dudit utilisateur (14) lorsque l'unité d'analyse (20) a détecté que le signal de variation FC dans le second mode de détection n'est pas dans la plage FC de référence.

2. Système (34) selon la revendication 1, dans lequel ladite unité de déclenchement (26) est conçue pour déclencher un processus de mesure du signal ECG lorsque ledit signal de variation FC dérivé s'écarte de ladite plage de FC de référence de plus d'une quantité prédéterminée.

3. Système (34) selon la revendication 2, dans lequel la quantité prédéterminée dépend des informations démographiques de l'utilisateur et/ou des antécédents médicaux de l'utilisateur et/ou d'au moins un signal physiologique de l'utilisateur et/ou de l'activité, laquelle l'utilisateur effectue.

4. Système (34) selon la revendication 1, dans lequel ladite unité optique de détection (12) comprend au moins un capteur de photopléthysmographie (PPG), ledit signal optique lié au rythme cardiaque comprenant un signal PPG.

5. Système (34) selon la revendication 1, dans lequel l'unité de déclenchement (26) déclenche les éléments suivants :
- le démarrage automatique d'une mesure ECG ; et/ou
- le déclenchement automatique d'un enregistrement de la mesure ECG ; et/ou
- le marquage des données de mesure ECG, lesquelles sont enregistrées ; et/ou
- la fourniture de l'information de l'utilisateur qu'il doit se connecter aux électrodes d'un appareil ECG ; et/ou
- l'affichage du signal ECG sur un élément d'interface utilisateur graphique (46).

6. Système (34) selon la revendication 1, comprenant en outre un élément d'interface utilisateur (46).

7. Système (34) selon la revendication 1, comprenant une unité électrique de détection (28) destinée à la mesure d'un signal électrique lié au rythme cardiaque dudit utilisateur (14).

8. Système (34) selon la revendication 7, dans lequel ladite unité électrique de détection comprend une unité ECG d'électrocardiographie comprenant au moins une première électrode (30) et une seconde électrode (32), ledit signal électrique lié au rythme cardiaque comprenant un signal ECG.

9. Système (34) selon la revendication 8, comprenant un dispositif de type montre-bracelet (10), ledit dispositif (10) comprenant un boîtier (38) pouvant être porté sur un poignet ou un bras dudit utilisateur (14).

10. Système (34) selon la revendication 9, dans lequel ladite première électrode (30) est disposée sur une première surface (42) dudit boîtier (38) en contact avec ledit poignet ou bras portant ledit boîtier (38) et ladite seconde électrode est disposée sur une seconde surface (44) dudit boîtier (38) ou et connectée audit boîtier (38) par l'intermédiaire d'un moyen de connexion (48).

11. Système (34) selon la revendication 1, dans lequel ledit dispositif (10) comprend un dispositif de communication (50).

12. Procédé de détection de variation de fréquence cardiaque, FC, d'un utilisateur (14), comprenant les étapes suivantes :
- la mesure d'un signal optique lié au rythme cardiaque dudit utilisateur (14) au cours du temps à l'aide d'une unité optique de détection (12) dans un premier mode de détection à une première fréquence d'échantillonnage ;
- la dérivée d'un signal de variation de fréquence cardiaque à partir dudit signal optique lié au rythme cardiaque ;
- la comparaison dudit signal de variation FC dérivé à une plage de FC de référence ;
- le passage du premier mode de détection, dans lequel l'unité optique de détection (12) a déjà effectué une mesure optique de l'utilisateur (14), à un second mode de détection à une seconde fréquence d'échantillonnage, laquelle correspond à une fréquence d'échantillonnage supérieure à la première fréquence d'échantillonnage lorsque la comparaison du signal de variation FC dans le premier mode de détection n'est pas dans la plage FC de référence ; et
- le déclenchement d'un processus de mesure d'un signal ECG dudit utilisateur (14) lorsque le signal de variation FC dans le second mode de détection n'est pas dans la plage FC de référence.

13. Programme informatique comprenant un moyen de code de programme destiné à l'amenée du système selon l'une quelconque des revendications 1 à 11 à mettre en œuvre les étapes du procédé selon la revendication 12 lorsque ledit programme informatique est exécuté sur le système.
